# EUROPÄISCHE PATENTSCHRIFT

(11) **EP 3 135 253 B1**
(45) Veröffentlichungstag und Bekanntmachung des Hinweises auf die Patenterteilung: **18.04.2018**
(21) Anmeldenummer: 15183120.3
(22) Anmeldetag: 31.08.2015
(51) Int. Cl.: A61F 2/38, A61B 17/72, A61F 2/28, A61F 2/30, A61F 2/48

(54) **IMPLANTIERBARE PROTHESE ZUM ERSATZ DES MENSCHLICHEN KNIEGELENKS**
IMPLANTABLE PROSTHESIS FOR REPLACING THE HUMAN KNEE JOINT
PROTHESE IMPLANTABLE DESTINEE AU REMPLACEMENT DE L'ARTICULATION DU GENOU HUMAIN

(43) Veröffentlichungstag der Anmeldung: 01.03.2017
(73) Patentinhaber: Baumgart, Rainer, 80539 München (DE)
(72) Erfinder: Baumgart, Rainer, 80539 München (DE)
(74) Vertreter: Sloboshanin, Sergej

(56) Entgegenhaltungen:
- EP-A1- 1 371 346
- EP-A2- 1 216 669
- WO-A2-2008/000496
- JP-A- 2011 139 785

## Beschreibung

Die Erfindung betrifft eine implantierbare Prothese zum Ersatz des menschlichen Kniegelenks, sowie ggf. auch der angrenzenden Knochenabschnitte, wobei die Prothese ein femorales und eine tibiales Prothesenelement aufweist, die zur Ausführung einer Kniebeugung mittels einer Gelenkeinrichtung gelenkig miteinander verbunden sind, wobei das tibiale Prothesenelement eine Durchgangsöffnung zur Durchführung eines Werkzeuges, eines stabförmigen Schaftverankerungsteils oder eines Verlängerungsmarknagels aufweist. Die Prothese wird vorzugsweise nach Resektion eines Knochentumors eingesetzt.

Bösartige Knochentumore sind häufig kniegelenksnah am Femur oder an der Tibia lokalisiert, so dass nach Entfernung des betroffenen Knochenabschnittes ein Teil des Schaftes als auch der dazu gehörige femorale oder tibiale Gelenkanteil durch künstliche Prothesenelemente ersetzt werden müssen.

In jedem Fall muss auch in den korrespondierenden Knochen ein Prothesenteil implantiert werden, um eine Artikulation und eine Kopplung mit dem Prothesenelement des betroffenen Knochens zu erreichen. Die Kopplung erfolgt durch eine Gelenkeinrichtung, wie z.B. einen Scharnier- oder Kugelmechanismus, die eine Beweglichkeit zwischen Femur und Tibia ermöglichen soll, die der natürlichen Beweglichkeit des Kniegelenks möglichst nahe kommt. Die Verankerung der Prothese im Femur und der Tibia erfolgt jeweils durch eine in den Knochen eingebrachte Schaftverankerung. Falls eine spätere Entfernung vorgesehen ist, kann die Schaftverankerung mit glatter Oberfläche ausgeführt sein, so dass kein Knochen einwächst. Falls eine definitive Verankerung vorgesehen ist, kann die Oberfläche aufgeraut oder beschichtet sein, so dass Knochengewebe einwachsen kann. Alternativ kann die Schaftverankerung auch mit Knochenzement fixiert werden.

Da bösartige Knochentumore häufig im Wachstumsalter auftreten, kommt es im weiteren Verlauf nach der chirurgischen Entfernung des Tumors und der Implantation einer Tumorprothese durch den Wegfall der Wachstumsfuge auf der betroffenen Seite und durch das weitere Wachstum der Gegenseite zu einer Beinlängendifferenz. Das verzögerte Wachstum betrifft zwar in erster Linie den von dem Tumor selbst befallenen aber auch den korrespondierenden Knochen, d.h. wenn der Tumor z.B. am Femur lokalisiert war, bleibt nicht nur der Femur sondern auch die Tibia im Wachstum zurück. Selbst wenn in diesem Fall der Schaftverankerungsteil der Tibia glatt und poliert ausgeführt ist, so dass der Knochen grundsätzlich weiter wachsen kann, kommt es somit auch in dem korrespondierenden Knochen zu einer Wachstumsverzögerung, die zu der entstehenden Beinlängendifferenz beiträgt.

Möglichkeiten den von dem Tumor befallenen Knochen zu verlängern sind aus der EP 1 371 346 A1 und der EP 2 468 216 A1 bekannt. In der EP 1 371 346 A1 wird hierzu durch die Prothese in den verbliebenen Knochen ein Austausch der Schaftverankerung gegen einen Verlängerungsmarknagel offenbart, der nach einer Osteotomie die beiden Knochenfragmente langsam auseinanderzieht, so dass in dem sich vergrößernden Spalt neues Knochengewebe entstehen kann (Kallusdistraktionsmethode). Bei Lokalisation im proximalen Femur erfolgt der Austausch ebenfalls von proximal, bei Lokalisation im distalen Femur erfolgt der Austausch ebenfalls von distal, wobei anatomisch bedingt nicht zwangsläufig eine Entkopplung der Prothese notwendig ist, da die Eintrittsstelle bei Kniebeugung axial für Werkzeuge und die Schaftverankerungsteile oder auch einen Distraktionsmarknagel erreichbar ist. Bei Lokalisation des Tumors in der proximalen Tibia ist bedingt durch die Überdeckung des axialen Zugangs durch das femorale Prothesenelement sowohl bei Streckung als auch bei Beugung ein Austausch der tibialen Verankerung ohne Entkopplung der Prothesenelemente nicht möglich.

Die EP 2 468 216 A1 offenbar erstmals eine Lösung, am Femur einen Austausch der Schaftverankerung in minimal invasiver Operationstechnik, indem von dem gegenüberliegenden Knochenende der Wechsel der Komponenten erfolgt. Sofern der Tumor am Femur lokalisiert ist und keine Längendifferenz am Unterschenkel besteht, kann somit mit einer Prothese der in EP 2 468 216 A1 beschriebenen Art ein erneuter großer Zugang über die Prothese vermieden und damit das Infektionsrisiko deutlich gesenkt werden.
Um bei Verlängerungsbedarf der Tibia, sei es nach primären Tumorbefall der Tibia oder wenn nach Tumorlokalisation am Femur auch die Tibia verkürzt ist und deshalb die vorläufige Schaftverankerung in der Tibia gegen einen Verlängerungsmarknagel und nach Wachstumsende der Verlängerungsmarknagel gegen eine beschichtete Schaftverankerung ausgetauscht werden soll, die in den Knochen dauerhaft einwächst, muss jedoch weiterhin jedes Mal die Prothese entkoppelt werden, wozu ein großer operativer Zugang mit einem erheblichen Infektionsrisiko erforderlich ist.
Der in der EP 2 468 216 A1 beschriebene Vorteil kommt somit letztendlich nur dann zum Tragen, wenn nicht gleichzeitig auch eine Unterschenkelverkürzung vorliegt, da die in der EP 2 468 216 A1 beschriebene Lösung am Unterschenkel nicht anwendbar ist. Für den häufigen Fall, dass die Verkürzung aber auch, so wie bereits erwähnt, den Unterschenkel betrifft, besteht bisher keine Möglichkeit, eine Verlängerung nach der in EP 1 371 346 A1 beschriebenen Art ohne Entkopplung der Prothese in minimal invasiver Technik durchführen zu können. Somit steht den vorteilhaften Möglichkeiten eines Beinlängenangleichs an die Gegenseite durch Knochenwachstum weiterhin das erhebliche Infektionsrisiko durch wiederholte große operative Zugänge im Bereich der Prothese gegenüber.

Die Präambel des Anspruchs 1 basiert auf der WO-A-2008/000496. Der Erfindung liegt deshalb die Aufgabe zugrunde, unter Beibehaltung der Vorteile der Knochenverlängerung nach der Kallusdistraktionsmethode das Ausmaß der erneuten operativen Eingriffe und damit das Infektionsrisiko auch für die Verlängerung der Tibia zu verringern.
Diese Aufgabe wird durch eine implantierbare Prothese mit den Merkmalen des Patentanspruchs 1 gelöst. Vorteilhafte Weiterbildungen der erfindungsgemäßen Prothese sind Gegenstand der Patentansprüche 2 bis 14.
Bei einer bevorzugten Ausführungsform der Erfindung weisen sowohl das tibiale Prothesenelement als auch das femorale Prothesenelement Durchgangsöffnungen und die Gelenkeinrichtung eine Passagemöglichkeit auf, durch die sowohl Werkzeuge, z.B. ein Knochenfräser, als auch ein Verlängerungsmarknagel oder eine glatte oder beschichtete Schaftverankerung von proximal in den Tibiaschaft eingebracht werden können. Die Passage durch die Gelenkeinrichtung, mit der die beiden Prothesenelemente miteinander gelenkig verbunden sind, ist so ausgebildet ist, dass ein mit der Durchgangsöffnung fluchtender und mit dieser in Verbindung stehender Führungskanal durch sie hindurch oder an ihr vorbei geht, wenn das tibiale Prothesenelement und das femorale Prothesenelement in einer einer Kniebeugung entsprechenden Funktionsstellung in einem vorherbestimmten Winkel zueinander angeordnet sind. Der Winkel ist so bestimmt, dass sowohl verschiedene Werkzeuge als auch ein Verlängerungsmarknagel oder eine Schaftverankerung über einen kleinen suprapatellaren Hautschnitt von ca. 2 cm Länge in den Tibiaschaft eingebracht werden können.

Da das femorale Prothesenelement vor Übergang in den Schaft eine ausreichende Breite aufweist, führt der Führungskanal nicht zu einer übermäßigen Schwächung und kann im Bereich des patellaren Gleitlagers bündig und stufenfrei zur Oberfläche mit einem Kunststoffeinsatz abgedeckt werden.

Um die Durchgangsöffnung im tibialen Prothesenelement in Flucht mit dem Führungskanal im femoralen Prothesenelement auszurichten, ist eine Kniebeugung von 20° bis 90°, vorzugsweise 30° bis 60°, besonders bevorzugt 30° bis 45° erforderlich. Die Gelenkeinrichtung zwischen dem femoralen und dem tibialen Prothesenelement ist entweder konstruktiv so gestaltet, dass sich bei der bestimmungsmäßigen Beugung die Durchgangsöffnung von alleine fluchtend ausrichtet oder die Ausrichtung erfolgt durch einen Werkzeugeingriff über den suprapatellaren Zugang oder eine zusätzliche Stichinzision, in dem eine vorübergehende Blockade der Gelenkeinrichtung herbei geführt wird.

Vorzugsweise umfasst die Gelenkeinrichtung einen mit dem femoralen Prothesenelement verbundenen Gelenkkopf mit einem Innenraum, in dem ein mit dem tibialen Prothesenelement verbundenes Gelenkelement zur Führung der Kniebeugung schwenkbar und ggf. auch geringfügig drehbar geführt ist, wobei in dem Gelenkkopf ein in den Innenraum mündender Durchgang und in dem Gelenkelement eine Passagemöglichkeit, z.B. eine Durchgangsbohrung ausgebildet sind, die in der Funktionsstellung zur Bildung eines Führungskanals mit dem Durchgang in dem tibialen Prothesenelement miteinander fluchten.

Das femorale Prothesenelement kann modular aus einem unterschiedlich langen Schaftersatz, wenn der Tumor am Femur lokalisiert war, und einem Gelenkkopf bestehen oder aber auch ein Teil bilden. Das tibiale Prothesenelement kann ebenso modular aus einem unterschiedlich langen Schaftersatz, wenn der Tumor an der Tibia lokalisiert war und dem Gelenkelement bestehen oder ein Teil bilden.

Über einen suprapatellaren Zugang kann eine Führungshülse eingebracht werden, was den Wechsel von Werkzeugen, Distraktionsmarknägeln oder Schaftverankerungen gewebeschonend erleichtert. Vorteilhaft kann die Führungshülse auch formschlüssig in das femorale Prothesenelement platziert und mit einem Konus, einem Bajonett oder einem Gewinde fixiert werden.

Um die Positionen des Durchgangs und der Durchgangsbohrung für den Wechsel zu sichern und das Kniegelenk in dieser Position zu stabilisieren, kann die Hülse auch weiter bis in die Gelenkeinrichtung oder auch bis in das tibiale Prothesenelement platziert und dort vorteilhaft mit einem Konus, einem Bajonett oder einem Gewinde kraft- oder formschlüssig fixiert werden.

Sofern die Gelenkeinrichtung als Scharnier mit einer Scharnierachse ausgebildet ist, kann die Scharnierachse entweder zweigeteilt oder im mittleren Bereich eine ausreichend dimensionierte Bohrung oder eine Ausnehmung aufweisen. Sofern es sich um eine Gelenkeinrichtung handelt, die mit dem femoralen oder tibialen Prothesenelement verbunden ist und z.B. die Form einer Kugel aufweist, kann der Führungskanal zentral oder dezentral durch die Gelenkeinrichtung, z.B. durch die Kugel und deren Befestigungselemente oder daran vorbei verlaufen.

Vorzugsweise wird in dem tibialen Prothesenelement so wie bereits in EP 2 468 216 A1 offenbart eine in den Hohlraum mündende Querbohrung vorgesehen, durch die ein Kabel oder ein Schlauch durchgeführt werden kann, das/der mit dem einen Ende an einer Energieeinspeisung, z.B. einer subkutanen Antenne und an dem anderen Ende mit dem Antrieb eines Distraktionsmarknagels verbindbar ist, wenn dieser nach Entfernen des Schaftverankerungsteils in das tibiale Prothesenelement eingeführt wird, um eine Knochendistraktion nach der Kallusdistraktionsmethode auszuführen.

Das Schaftverankerungsteil kann ein massiver, glatter oder beschichteter Stabilisator oder ein Marknagel mit integriertem Antrieb für eine Distraktion des Knochens sein.

Nach Resektion eines Tumors am distalen Femur bei Kindern im Wachstumsalter wird zunächst das femorale Prothesenelement, das je nach Größe des Tumors den Schaft partiell ersetzt, implantiert. Das Prothesenelement kann je nach erforderlicher Länge aus einer Komponente oder auch modular aus mehreren Komponenten bestehen. An der Tibia wird das korrespondierende Prothesenelement implantiert indem zunächst ein polierter Schaft durch die Durchgangsöffnung in den Tibiaschaft eingebracht wird, so dass weiteres Wachstum möglich ist.

Nach Resektion eines Tumors an der proximalen Tibia bei Kindern im Wachstumsalter wird zunächst das tibiale Prothesenelement, das ebenfalls je nach Größe des Tumors den Schaft partiell ersetzt, implantiert, wobei das Prothesenelement auch hier je nach erforderlicher Länge aus einer Komponente oder auch modular aus mehreren Komponenten bestehen kann. Am Femur wird das korrespondierende Prothesenelement implantiert indem auch hier zunächst ein polierter Schaft eingebracht wird, so dass weiteres Wachstum hier möglich ist.

Bei Verlängerungsbedarf aufgrund des Wachstums der Gegenseite kann nun gemäß dem Stand der Technik, so wie in den Patenten 1 371 346 A1 oder Patent EP 2 468 216 A1 offenbart am Femur der Austausch der Schaftverankerung gegen einen Distraktionsmarknagel ohne eine Entkopplung der Prothese vorgenommen werden. Bei dem bestimmungsgemäßen Gebrauch der vorliegenden Anmeldung kann nun ebenfalls ohne eine Entkopplung der Prothese die vorläufige Schaftverankerung des tibialen Prothesenelements ausgetauscht und ggf. z.B. durch einen Distraktionsmarknagel ersetzt werden.

Hierzu wird das Kniegelenk in einen vorbestimmten Winkel positioniert, der, ausgehend von einer gestreckten Stellung, vorzugsweise etwa zwischen 30°und 45° liegt. Über einen kleinen Hautschnitt oberhalb der Patella erfolgt nach Längsinzision der Quadrizepssehne das Einbringen eines Dilatators, der einen Tunnel durch die Weichteile zum femoralen Prothesenelement bahnt. Über den Dilatator kann nun vorzugsweise eine Führungshülse vorgeschoben werden, die in dem femoralen Prothesenelement durch Form- oder Kraftschluss verankert werden kann. Sofern sich der Kopplungsmechanismus nicht automatisch fluchtend ausrichtet, erfolgt nun die Ausrichtung und ggf. auch die vorübergehende Fixierung zwischen dem femoralen und tibialen Prothesenelement und ggf. auch mit der Gelenkeinrichtung entweder unter optischer Kontrolle unter Bildwandler oder mit einem geeigneten Werkzeug über eine Stichinzision, z.B. mit einem Metallstift. Um die Funktionsstellung des Kniegelenks zu fixieren, kann die Führungshülse bei weiteren Ausführungsformen auch durch das femorale Prothesenelement hindurch bis in die Gelenkeinrichtung oder auch weiter bis in das tibiale Prothesenelement vorgeschoben werden. Über das so geschaffene Portal können nun zunächst die vorläufige Schaftverankerung des tibialen Prothesenelements entfernt und dann Fräswerkzeuge zum Einsatz kommen oder auch ein Distraktionsmarknagel implantiert werden. Auf gleiche minimal invasive Weise können Distraktionsmarknägel bei wiederholtem Verlängerungsbedarf ausgetauscht und am Ende der Verlängerungsphase gegen eine definitive, vorzugsweise beschichtete Schaftverankerung ersetzt werden.

Anhand von Zeichnungen wird die Erfindung näher erläutert. Es zeigen
- Fig. 1: in einem Querschnitt in der Sagittalebene schematisch eine ersten Ausführungsform einer implantierbaren Prothese zum Ersatz eines menschlichen Kniegelenks,
- Fig. 2: in einer detaillierteren Teilansicht die Ausbildung der Prothese von Fig. 1,
- Fig. 3: in einem Querschnitt in der Sagittalebene schematisch die Prothese von Fig. 1 mit einer in eine Gelenkeinrichtung eingesetzten Führungshülse, durch die ein Werkzeug, z.B. ein Fräser eingeführt wird,
- Fig. 4: die Ausführungsform der Prothese von Fig. 1 mit einem in einem Durchgang des femoralen Gelenkkopfes angeordneten Verschlussstopfen,
- Fig. 5: in einem Querschnitt in der Sagittalebene schematisch eine zweite Ausführungsform einer implantierbare Prothese zum Ersatz eines menschlichen Kniegelenks mit einem in der Tibia angeordneten Verlängerungsmarknagel,
- Fig. 6: in einem Querschnitt in der Sagittalebene schematisch eine dritte Ausführungsform einer implantierbaren Prothese zum Ersatz eines menschlichen Kniegelenks mit einer nur in dem femoralen Gelenkkopf eingesetzten Führungshülse.

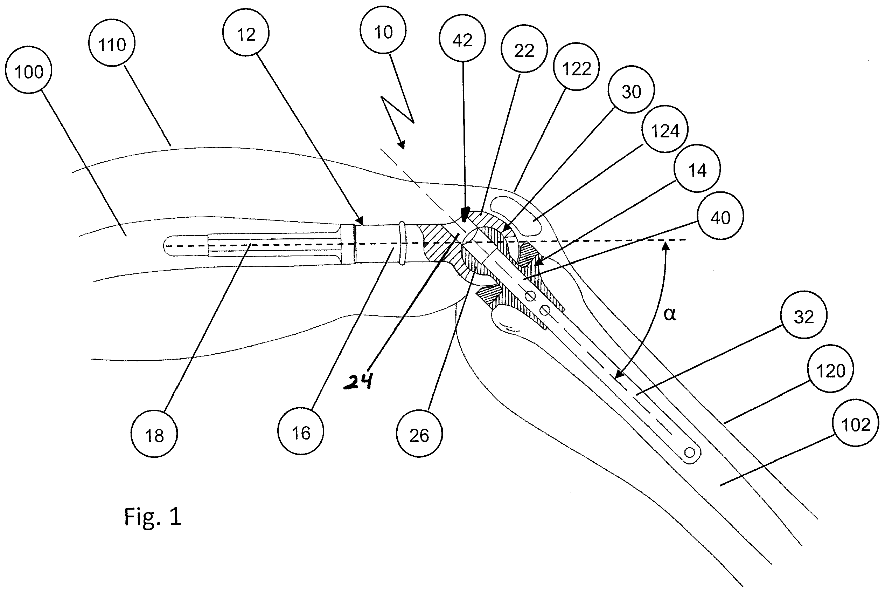

Figur 1 zeigt im Querschnitt in der Sagittalebene schematisch eine erste Ausführungsform einer implantierten Prothese 10 zum Ersatz eines menschlichen Kniegelenks. Die Prothese 10 umfasst ein femorales Prothesenelement 12, das mittels eines femoralen Schaftverankerungsteils 18 im Femur 100 eines Oberschenkels 110 einer Person verankert ist.

Das femorale Prothesenelement 12 wird nach Resektion eines Tumors am distalen Femur 100 implantiert, wobei je nach Größe des Tumors der Schaft des Femur 100 durch einen femoralen Schaftersatz 16 partiell ersetzt wird. Das Prothesenelement 12 kann je nach erforderlicher Länge aus einer Komponente oder modular aus mehreren Komponenten bestehen.

Ein tibiales Prothesenelement 14 ist mittels eines tibialen Schaftverankerungsteils 32 in der Tibia 102 des Unterschenkels 120 verankert und kann ebenfalls modular aufgebaut oder nur aus einem Teil bestehen. Die Prothesenelemente 12, 14 sind mittels einer Gelenkeinrichtung 30 so gelenkig miteinander verbunden, dass eine einer Kniebeugung entsprechende Bewegung des Unterschenkels 120 bezüglich des Oberschenkels 110 ermöglicht ist.

Die Gelenkeinrichtung 30 umfasst einen an dem distalen Ende des femoralen Prothesenelements 12 angebrachten Gelenkkopf 22, in dem ein Gelenkelement 26 drehbar gelagert ist, das mit dem proximalen Ende des tibialen Prothesenelements 14 verbunden ist.

Der Gelenkkopf 22 kann mit dem femoralen Prothesenelement 12 durch Verschraubung, Verschweißung, Verklebung oder ähnlichem verbunden werden. Auf gleiche Weise kann das Gelenkelement 26 mit dem Prothesenelement 14 verbunden werden. Das Prothesenelement 12 und der Gelenkkopf 22 können jedoch auch als ein Teil aus einem Stück gefertigt werden, ebenso wie das Gelenkelement 26 und das Prothesenelement 14.

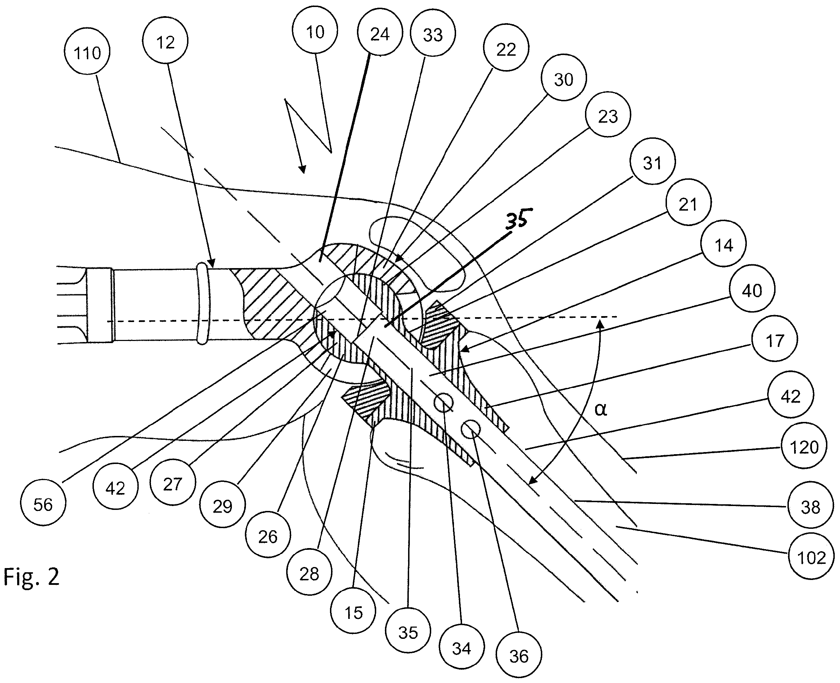

Die Ausbildung der Prothese 10 von Figur 1 ist in Figur 2 detaillierter gezeigt. Der Unterschenkel 102 ist in der in Fig. 1 gezeigten Funktionsstellung bezüglich des Oberschenkels 110 um einen Winkel α aus einer gestreckten Stellung des Knies nach unten gebeugt. Der Winkel α beträgt 20° bis 90°, vorzugsweise 30° bis 60°. Bei einer besonders bevorzugten Ausführungsform beträgt der Winkel α 30° bis 45°.

In dem Gelenkkopf 22 ist ein zylindrischer Innenraum 23 ausgebildet, der über einen Durchgang 24 mit kreisförmigem Querschnitt schräg nach oben in Richtung der Oberseite des Oberschenkels 110 mündet, wobei der Winkel zwischen der Mittelachse des Durchgangs 24 und der Mittelachse des Prothesenelements 12 im Wesentlichen dem Winkel α entspricht. In dem Innenraum 23 ist das Gelenkelement 26 mit einem zylindrischen Kopfabschnitt 27 schwenkbar gelagert. Von dem Kopfabschnitt 27 erstreckt sich ein Fußabschnitt 21 durch eine Aussparung 29 des Gelenkkopfs 22 in Richtung des tibialen Prothesenelements 14. Die Aussparung 29 ist bezüglich der Mittelachse des Kopfabschnitts 27 und des Innenraums 23 dem Durchgang 24 diametral gegenüberliegend ausgebildet und so dimensioniert, dass eine für alle Kniebeugungen ausreichende Verschwenkung des Gelenkelements 26 möglich ist, ohne dass eine Bewegung des Fußabschnitts 21 in der Aussparung 29 behindert wird.

Das tibiale Prothesenelement 14 ist in das gelenknahe, proximale Ende der Tibia 102 mit einem sich in distaler Richtung verjüngenden Fixierfortsatz 17 eingesetzt und fixiert, und umfasst einen Kragenabschnitt 15, der nach dem Einsetzen des Prothesenelements 14 auf dem proximalen Ende der Tibia 102 aufliegt. Das distale Stirnende des Fußabschnitts 21 des Gelenkelements 26 ist mit dem tibialen Prothesenelement 14 fest verbunden und weist eine zentrale Durchgangsöffnung 40 mit kreisförmigem Querschnitt auf, die in der Funktionsstellung koaxial zu dem Durchgang 24 durch den Kragenabschnitt 15 und den Fixierfortsatz 17 verläuft. Durch das Gelenkelement 26 geht eine Durchgangsbohrung 28 mit kreisförmigem Querschnitt hindurch, die in der Funktionsstellung ebenfalls koaxial zu dem Durchgang 24 verläuft.

Der Durchgang 24 und die Durchgangsbohrung 28 bilden in der in den Fig.1 und 2 gezeigten Funktionsstellung zusammen einen Führungskanal 42 mit kreisförmigem Querschnitt, der mit der Durchgangsöffnung 40 in dem Prothesenelement 14 in Verbindung steht. Die Durchgangsbohrung 28 hat einen distalen Abschnitt 35, der mit der Durchgangsöffnung 40 verbunden ist und den gleichen Durchmesser wie diese aufweist. An diesen distalen Abschnitt 35 schließt in vorteilhafter Weise ein proximaler erweiterter Abschnitt 56 der Durchgangsbohrung 28 an, der in der in Figur 2 gezeigten Funktionsstellung mit dem Durchgang 24 fluchtend in Verbindung steht. Hierdurch wird zwischen dem erweiterten Abschnitt 56 und dem gelenkfernen Abschnitt 35 eine Stufe 33 in dem Gelenkelement 26 gebildet.

Im Bereich des Fixierfortsatzes 17 sind in dem Prothesenelement 14 in axialer Richtung im Abstand zueinander angeordnete Querbohrungen 34, 36 vorgesehen, die durch die Wandung des Fixierfortsatzes 17 hindurchgehen. Durch die Querbohrungen 34, 36 können Befestigungsstifte zur Befestigung eines tibialen Schaftverankerungsteils 32 oder eines Verlängerungsmarknagels 80, sowohl zum Fixierungsfortsatz 17 als auch zur Tibia 102 durchgeführt werden.

Auf der proximalen Oberfläche des Kragenabschnitts 15 ist ein Gleitblock 31 angebracht, auf dem der Gelenkkopf 22 bei einer Kniebewegung zur Vermeidung von Verschleiß gleitet.

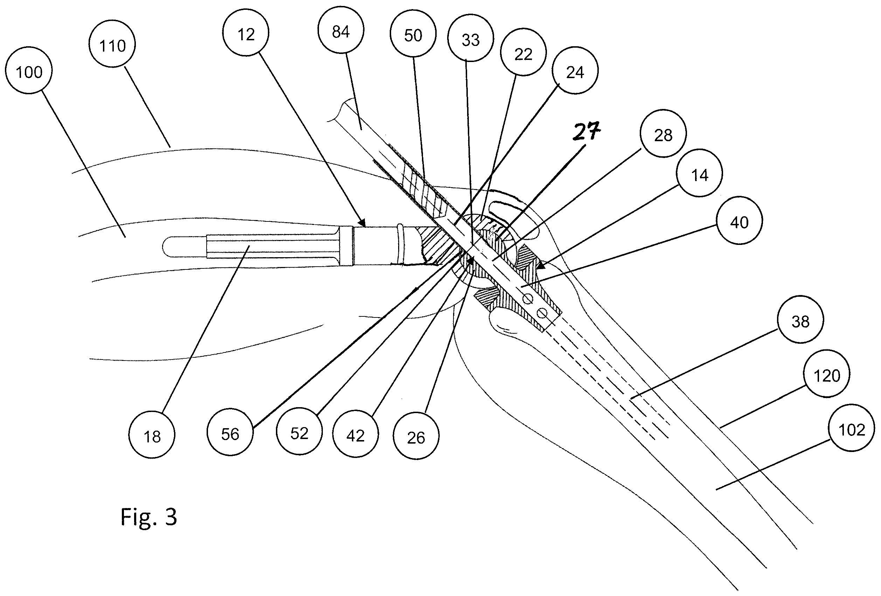

In Figur 3 ist die Einführung einer Führungshülse 50 durch den Durchgang 24 in den erweiterten Abschnitt 56 der Durchgangsbohrung 28 gezeigt. Die Durchgangsbohrung 28 und die Durchgangsöffnung 40 sind immer koaxial zueinander angeordnet, da das Gelenkelement 26 fest mit dem Prothesenelement 14 verbunden ist oder mit diesem ein Teil bildet. Wenn der Unterschenkel 120 bezüglich des Oberschenkels 110 aus einer gestreckten Kniestellung um den Winkel α (siehe Figur 2) in die Funktionsstellung verschwenkt wird, fluchtet der Durchgang 24 in dem Gelenkkopf 22 mit der Durchgangsöffnung 40 und der Durchgangsbohrung 28 und bildet mit der Durchgangsbohrung 28 den Führungskanal 42. In dieser Stellung wird eine Führungshülse 50 über einen kleinen Hautschnitt nach Längsinzision der Quadrizepssehne über einen Dilatator (nicht gezeigt) eingebracht und mit einem Eingriffsende 52 durch den Durchgang 24 hindurch in den erweiterten Abschnitt 56 der Durchgangsbohrung 28 eingeschoben, bis das Eingriffsende 52 auf der Stufe 33 aufsetzt. Der Außendurchmesser der Führungshülse 50 entspricht dem Innendurchmesser des erweiterten Abschnitts 56. Der Innendurchmesser der Führungshülse 50 ist gleich dem Innendurchmesser des distalen Abschnitts 35 und der Durchgangsöffnung 40.

Durch das Einführen des Eingriffsendes 52 der Führungshülse 50 in den erweiterten Abschnitt 56 in dem Gelenkelement 26 wird eine Drehung des Gelenkelements 26 bezüglich des Gelenkkopfs 22 verhindert. Wie es in Figur 3 gezeigt ist, kann nun ein Werkzeug, z.B. ein Fräser 84, dessen Außendurchmesser dem Innendurchmesser der Führungshülse 50 entspricht, zur Ausbildung eines Bohrkanals 38 in der Tibia 102 durch den Durchgang 24, die Durchgangsbohrung 28 und die Durchgangsöffnung 40 hindurch geführt werden. Durch die Führungshülse können auch andere Werkzeuge, wie z.B. Bohrer, oder Schaftverankerungsteile oder Verlängerungsmarknägel eingeführt oder entfernt werden.

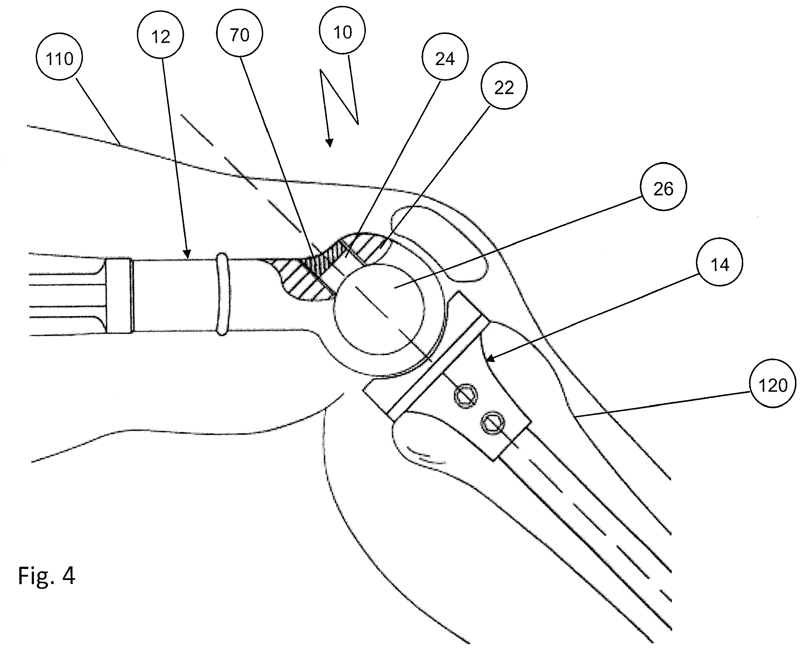

In Figur 4 ist gezeigt, dass der Durchgang 24 nach der Verwendung eines Werkzeug oder nach Einführung oder Entfernung eines Verlängerungsmarknagels oder eines Schaftverankerungsteils durch einen Verschlussstopfen 70 verschlossen werden kann, dessen Außenfläche bündig zur Oberfläche der femoralen Prothesenkomponente ist. Der Verschlussstopfen besteht vorzugsweise aus PTFP, PVC oder PEEK.

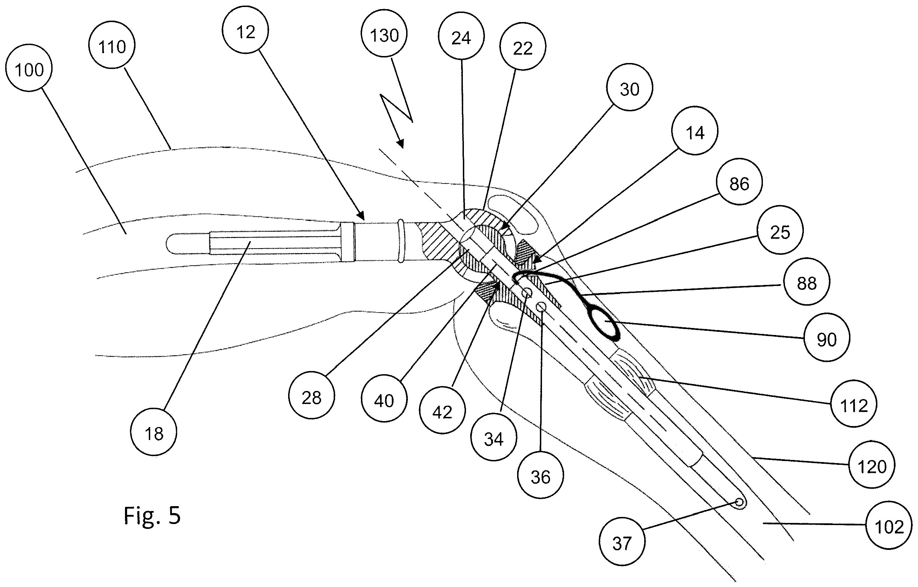

Die in Figur 5 gezeigte zweite Ausführungsform der Prothese 130 entspricht im Wesentlichen der Ausführungsform von Figur 1 bis 4 und unterscheidet sich im Wesentlichen nur dadurch, dass durch die Wandung des Fixierfortsatzes 25 des tibialen Prothesenelements 14 eine Seitenöffnung 86 hindurchgeht. Durch den Durchgang 24, die Durchgangsbohrung 28 und die Durchgangsöffnung 40 ist ein Verlängerungsmarknagel 80 hindurchgeführt, der mittels eines Motors verlängerbar ist, so dass eine Knochenverlängerung nach der Kallusdistraktionsmethode möglich ist und neues Knochengewebe 112 im Distraktionsspalt der durchtrennten Tibia entstehen kann. Zur Steuerung des Motors geht ein mit dem Verlängerungsmarknagel 80 verbundenes Kabel 88 durch die Seitenöffnung 86 nach außen hindurch. An dem freien Ende des Kabels 88 ist eine Antenne 90 vorgesehen. Der Verlängerungsmarknagel 80 wird durch durch die Querbohrungen 34, 36 hindurchgehende Bolzen an dem Prothesenelement 14 und möglicherweise auch an der proximalen Tibia und zusätzlich durch durch eine Querbohrung 37 hindurchgehenden Bolzen an der distalen Tibia fixiert.

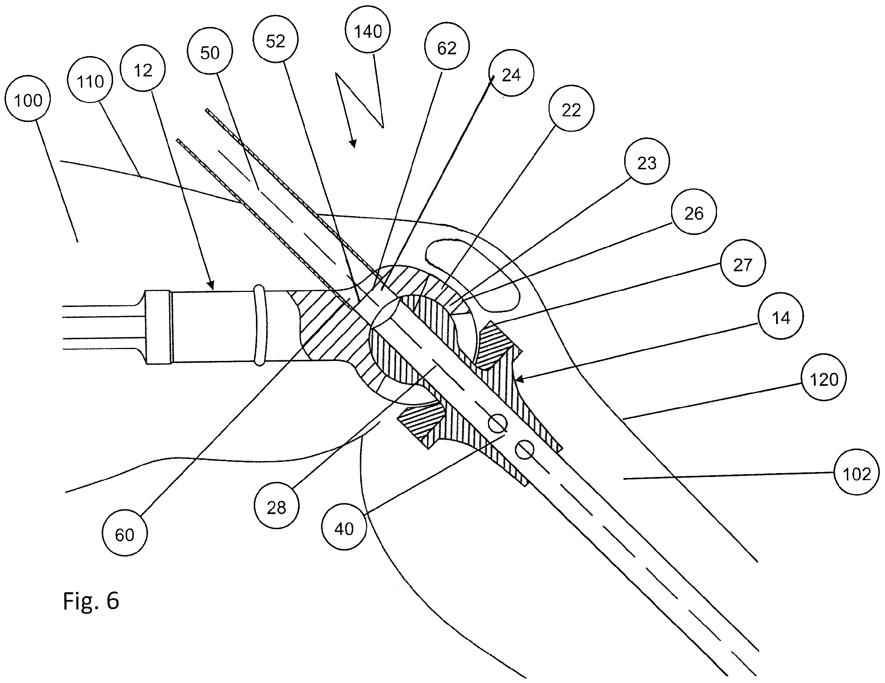

Die in Figur 6 gezeigte Ausführungsform der Prothese 140 unterscheidet sich von der in Figur 2 gezeigten Ausführungsform dadurch, dass der Durchgang 28 einen durchgehend konstanten Durchmesser aufweist, der dem Durchmesser der Durchgangsöffnung 40 entspricht. Der Durchgang 24 weist einen proximalen erweiterten Abschnitt 60 auf, der zur Außenseite des Gelenkkopfes 22 mündet. Hierdurch wird eine Stufe 62 gebildet. Eine Führungshülse 50 ist so eingesetzt, dass sie mit ihrem Eingriffsende 52 an der Stufe 62 aufliegt.

Bei den dargestellten Ausführungsformen weist der Gelenkkopf 22 einen zylindrischen Innenraum 23 auf und der Kopfabschnitt 27 des Gelenkelements 26 ist im distalen Bereich ebenfalls zylindrisch ausgebildet, wobei der Außendurchmesser des Kopfabschnitts 27 dem Durchmesser des Innenraums 23 entspricht, wodurch eine Scharnierverbindung gebildet wird. Es sind jedoch auch andere Gelenkverbindungen zwischen dem femoralen Prothesenelement 12 und dem tibialen Prothesenelement 14 möglich. Beispielsweise kann der Innenraum 23 sphärisch ausgebildet sein und der Kopfabschnitt 27 kugelförmig, um dreidimensionale Bewegungen zu ermöglichen.

Es ist auch nicht unbedingt erforderlich, dass der Führungskanal 42 durch die Gelenkeinrichtung 30 hindurchgeht. Grundsätzlich ist es auch denkbar, dass der Führungskanal 42 seitlich außerhalb der Gelenkeinrichtung 30 verläuft. Die Gelenkeinrichtung 30 kann auch zwei oder mehrere längs der Schwenkachse im Abstand angeordnete Elemente aufweisen. Der Führungskanal 42 könnte dann zwischen zweien dieser Elemente angeordnet werden.

### Bezugszeichenliste

10 Prothese
12 femorales Prothesenelement
14 tibiales Prothesenelement
15 Kragenabschnitt
16 femoraler Schaftersatz
17 Fixierfortsatz
18 femoraler Schaftverankerungsteil
21 Fußabschnitt
22 Gelenkkopf
23 Innenraum
24 Durchgang
25 Fixierfortsatz
26 Gelenkelement
27 Kopfabschnitt
28 Durchgangsbohrung
29 Aussparung
30 Gelenkeinrichtung
31 Gleitblock
32 tibialer Schaftverankerungsteil
33 Stufe
34 Querbohrung
35 gelenkferner Abschnitt
36 Querbohrung
37 Querbohrung
38 Bohrkanal
40 Durchgangsöffnung
42 Führungskanal
50 Führungshülse
52 Eingriffsende
56 erweiterter Abschnitt
60 erweiterter Abschnitt
62 Stufe
70 Verschlussstopfen
80 Verlängerungsmarknagel
84 Werkzeug
86 Seitenöffnung
88 Kabel
90 Antenne
100 Femur
102 Tibia
110 Oberschenkel
112 neues Knochengewebe
120 Unterschenkel
122 Knie
124 Patella
130 Prothese
140 Prothese

## Patentansprüche

1. Implantierbare Prothese zum Ersatz eines menschlichen Kniegelenks,
mit einem femoralen Prothesenelement (12) und einem tibialen Prothesenelement (14), die zur Ausführung einer Kniebeugung mittels einer Gelenkeinrichtung (30) gelenkig miteinander verbunden sind, wobei das tibiale Prothesenelement (14) eine Durchgangsöffnung (40) zur Durchführung eines Werkzeugs (84), eines stabförmigen tibialen Schaftverankerungsteils (32) oder eines Verlängerungsmarknagels (80) aufweist,
**dadurch gekennzeichnet, dass**
die Gelenkeinrichtung (30) so ausgebildet ist, dass sie einen Führungskanal (42) zur Durchführung des Werkzeugs (84), des stabförmigen tibialen Schaftverankerungsteils (32) oder des Verlängerungsmarknagels (80) von außen bildet, der mit der Durchgangsöffnung (40) fluchtet und mit dieser in Verbindung steht, wenn das tibiale Prothesenelement (14) und das femorale Prothesenelement (12) in einer einer Kniebeugung entsprechenden Funktionsstellung in einem vorherbestimmten Winkel zueinander angeordnet sind.

2. Prothese nach Anspruch 1, **dadurch gekennzeichnet, dass** der vorherbestimmte Winkel einer Kniebeugung von 20° bis 90°, vorzugsweise 30° bis 60°, besonders bevorzugt 30° bis 45° aus einer Streckstellung des Knies beträgt.

3. Prothese nach Anspruch 1 oder 2, **dadurch gekennzeichnet, dass** die Gelenkeinrichtung (30) einen mit dem femoralen Prothesenelement (12) verbundenen Gelenkkopf (22) mit einem Innenraum (23) umfasst, in dem ein mit dem tibialen Prothesenelement (14) verbundenes Gelenkelement (26) zur Führung der Kniebeugung drehbar geführt ist, wobei in dem Gelenkkopf (22) ein in den Innenraum (23) mündender Durchgang (24) und in dem Gelenkelement (26) eine Durchgangsbohrung (28) ausgebildet sind, die in der Funktionsstellung zur Bildung des Führungskanals (42) miteinander fluchten.

4. Prothese nach einem der Ansprüche 1 bis 3, **dadurch gekennzeichnet, dass** der Durchgang (24) und die Durchgangsbohrung (28) einen kreisförmigen Querschnitt und den gleichen Durchmesser aufweisen.

5. Prothese nach einem der Ansprüche 1 bis 3, **dadurch gekennzeichnet, dass** der Durchgang (24) und die Durchgangsbohrung (28) einen kreisförmigen Querschnitt aufweisen, wobei der Durchmesser des Durchgangs (24) an einem nach außen mündenden, erweiterten Abschnitt (60) größer ist als der Durchmesser der Durchgangsbohrung (28), so dass in den Durchgang (24) eine Führungshülse (50) einsetzbar ist, deren Innendurchmesser dem Innendurchmesser der Durchgangsbohrung (28) entspricht.

6. Prothese nach einem der Ansprüche 1 bis 3, **dadurch gekennzeichnet, dass** der Durchgang (24) und die Durchgangsbohrung (28) einen kreisförmigen Querschnitt aufweisen, wobei der Durchmesser des Durchgangs (24) größer ist als der Durchmesser der Durchgangsbohrung (28) und die Durchgangsbohrung (28) an ihrem, dem Durchgang (24) zugewandten Ende einen erweiterten Abschnitt (56) aufweist, dessen Durchmesser dem Durchmesser des Durchgangs (24) entspricht, so dass in den Durchgang (24) eine Führungshülse (50), deren Innendurchmesser dem Innendurchmesser der Durchgangsbohrung (28) entspricht, in der Funktionsstellung so einsetzbar ist, dass sie mit einem Eingriffsende (52) zur Blockierung einer unbeabsichtigten Gelenkbewegung in den erweiterten Abschnitt (56) eingreift.

7. Prothese nach Anspruch 5, **dadurch gekennzeichnet, dass** an der Führungshülse (50) und dem Durchgang (24) Mittel zur lösbaren Fixierung der Führungshülse (50) in dem Durchgang (24) vorgesehen sind.

8. Prothese nach Anspruch 6, **dadurch gekennzeichnet, dass** an der Führungshülse (50) und der Durchgangsbohrung (28) Mittel zur lösbaren Fixierung der Führungshülse (50) in der Durchgangsbohrung (28) vorgesehen sind.

9. Prothese nach einem der vorhergehenden Ansprüche 3 bis 8, **dadurch gekennzeichnet, dass** in dem Durchgang (24) ein Verschlussstopfen (70) lösbar angeordnet ist, dessen Außenfläche bündig zur Oberfläche der femoralen Prothesenkomponente ist.

10. Prothese nach Anspruch 9, **dadurch gekennzeichnet, dass** der Verschlussstopfen (70) aus PTFE, PVC oder PEEK besteht.

11. Prothese nach einem der vorhergehenden Ansprüche, **dadurch gekennzeichnet, dass** in dem tibialen Prothesenelement (14) wenigstens eine seitliche Querbohrung (34) ausgebildet ist, durch die Querschrauben oder Querbolzen zur formschlüssigen Verbindung des tibialen Prothesenelements (14) mit einem Schaftverankerungsteil (32), einem Verlängerungsmarknagel (80) und/oder der Tibia (102) einsetzbar sind.

12. Prothese nach einem der vorhergehenden Ansprüche, **dadurch gekennzeichnet, dass** in dem tibialen Prothesenelement (14) eine Seitenöffnung (86) zur Durchführung eines mit einem Verlängerungsmarknagel (80) verbindbaren Kabels (88) vorgesehen ist.

13. Prothese nach einem der vorhergehenden Ansprüche, **dadurch gekennzeichnet, dass** der Führungskanal (42) durch die Gelenkeinrichtung (30) hindurchgeht.

14. Prothese nach einem der Ansprüche 1 bis 12, **dadurch gekennzeichnet, dass** der Führungskanal (42) außerhalb der Gelenkeinrichtung (30) angeordnet ist.

15. Prothese nach einem der Ansprüche 3 bis 14, **dadurch gekennzeichnet, dass** das femorale Prothesenelement (12) mit dem Gelenkkopf (22) ein Teil bildet und/oder das tibiale Prothesenelement (14) mit dem Gelenkelement (26) ein Teil bildet.

## Claims

1. Implantable prosthesis for replacing a human knee joint,
comprising a femoral prosthetic element (12) and a tibial prosthetic element (14) which are articulated with one another by means of a joint device (30) for the purpose of executing a knee bend, the tibial prosthetic element (14) having a through-opening (40) for the passage of a tool (84), a rod-like tibial shaft anchoring part (32) or an intramedullary lengthening nail (80),
**characterized in that**
the joint device (30) is designed such that it forms a guide channel (42) for the passage of the tool (84), the rod-like tibial shaft anchoring part (32) or the intramedullary lengthening nail (80) from the outside, which guide channel is in alignment with the through-opening (40) and is in connection therewith when the tibial prosthetic element (14) and the femoral prosthetic element (12) are arranged at a predetermined angle relative to one another in a functional position corresponding to a knee bend.

2. Prosthesis according to claim 1, **characterized in that** the predetermined angle of a knee bend is from 20° to 90°, preferably from 30° to 60°, particularly preferably from 30° to 45°, from an extended position of the knee.

3. Prosthesis according to claim 1 or claim 2, **characterized in that** the joint device (30) comprises a joint head (22) which is connected to the femoral prosthetic element (12) and has an interior space (23) in which a joint element (26) connected to the tibial prosthetic element (14) is rotatably guided for guiding the knee bend, a passage (24) opening into the interior space (23) being formed in the joint head (22) and a through-bore (28) being formed in the joint element (26), the passage and the through-bore being in alignment with one another in the functional position to form the guide channel (42).

4. Prosthesis according to any of claims 1 to 3, **characterized in that** the passage (24) and the through-bore (28) have a circular cross section and the same diameter.

5. Prosthesis according to any of claims 1 to 3, **characterized in that** the passage (24) and the through-bore (28) have a circular cross section, the diameter of the passage (24) at an outwardly opening, widened portion (60) being larger than the diameter of the through-bore (28), so that a guide sleeve (50) whose inside diameter corresponds to the inside diameter of the through-bore (28) can be inserted into the passage (24).

6. Prosthesis according to any of claims 1 to 3, **characterized in that** the passage (24) and the through-bore (28) have a circular cross section, the diameter of the passage (24) being larger than the diameter of the through-bore (28) and the through-bore (28) having at its end facing the passage (24) a widened portion (56) whose diameter corresponds to the diameter of the passage (24), so that a guide sleeve (50) whose inside diameter corresponds to the inside diameter of the through-bore (28) can be inserted into the passage (24) in the functional position so that it engages in the widened portion (56) with an engagement end (52) to block an unintentional joint movement.

7. Prosthesis according to claim 5, **characterized in that** means for releasably fixing the guide sleeve (50) in the passage (24) are provided on the guide sleeve (50) and the passage (24).

8. Prosthesis according to claim 6, **characterized in that** means for releasably fixing the guide sleeve (50) in the through-bore (28) are provided on the guide sleeve (50) and the through-bore (28).

9. Prosthesis according to any of the preceding claims 3 to 8, **characterized in that** a plug (70) is releasably arranged in the passage (24), the outer surface of which plug is flush with the surface of the femoral prosthesis component.

10. Prosthesis according to claim 9, **characterized in that** the plug (70) is made of PTFE, PVC or PEEK.

11. Prosthesis according to any of the preceding claims, **characterized in that**, in the tibial prosthetic element (14), at least one lateral transverse bore (34) is formed through which transverse screws or transverse bolts for the interlocking connection of the tibial prosthetic element (14) to a shaft anchoring part (32), an intramedullary lengthening nail (80) and/or the tibia (102) can be inserted.

12. Prosthesis according to any of the preceding claims, **characterized in that**, in the tibial prosthetic element (14), a lateral opening (86) is provided for the passage of a cable (88) which can be connected to an intramedullary lengthening nail (80).

13. Prosthesis according to any of the preceding claims, **characterized in that** the guide channel (42) passes through the joint device (30).

14. Prosthesis according to any of claims 1 to 12, **characterized in that** the guide channel (42) is arranged outside the joint device (30).

15. Prosthesis according to any of claims 3 to 14, **characterized in that** the femoral prosthetic element (12) forms a part with the joint head (22) and/or the tibial prosthetic element (14) forms a part with the joint element (26).

## Revendications

1. Prothèse implantable pour le remplacement d'une articulation de genou humaine, avec un élément de prothèse fémoral (12) et un élément de prothèse tibial (14) qui sont reliés l'un à l'autre de manière articulée au moyen d'un dispositif d'articulation (30) pour l'exécution d'une flexion du genou, dans laquelle l'élément de prothèse tibial (14) comprend une ouverture traversante (40) pour la mise en oeuvre d'un instrument (84), d'une pièce d'ancrage de manche tibiale en forme de tige (32) ou d'un clou intramédullaire d'allongement (80),
**caractérisée en ce que**
le dispositif d'articulation (30) est conçu de telle sorte qu'il forme un canal de guidage (42) pour la mise en oeuvre de l'instrument (84), de la pièce d'ancrage de manche tibiale en forme de tige (32) ou du clou intramédullaire d'allongement (80) depuis l'extérieur, lequel canal de guidage (42) est aligné avec l'ouverture traversante (40) et est en liaison avec celle-ci, lorsque l'élément de prothèse tibial (14) et l'élément de prothèse fémoral (12) sont disposés l'un par rapport à l'autre selon un angle prédéterminé dans une position de fonctionnement correspondant à une flexion du genou.

2. Prothèse selon la revendication 1, **caractérisée en ce que** l'angle prédéterminé d'une flexion du genou va de 20° à 90°, de préférence de 30° à 60°, de manière particulièrement préférée de 30° à 45° à partir d'une position d'extension du genou.

3. Prothèse selon la revendication 1 ou la revendication 2, **caractérisée en ce que** le dispositif d'articulation (30) comporte une tête d'articulation (22) reliée à l'élément de prothèse fémoral (12), avec un espace intérieur (23), dans laquelle un élément d'articulation (26) relié à l'élément de prothèse tibial (14) est guidé rotatif pour l'exécution de la flexion du genou, dans laquelle un passage (24) débouchant dans l'espace intérieur (23) est ménagé dans la tête d'articulation (22) et un trou traversant (28) est ménagé dans l'élément d'articulation (26), lesquels sont alignés l'un avec l'autre dans la position de fonctionnement pour la formation du canal de guidage (42).

4. Prothèse selon l'une des revendications 1 à 3, **caractérisée en ce que** le passage (24) et le trou traversant (28) présentent une section transversale circulaire et le même diamètre.

5. Prothèse selon l'une des revendications 1 à 3, **caractérisée en ce que** le passage (24) et le trou traversant (28) présentent une section transversale circulaire, dans laquelle le diamètre du passage (24) sur une section élargie (60) débouchant vers l'extérieur est supérieur au diamètre du trou traversant (28), de telle sorte qu'une douille de guidage (50) dont le diamètre intérieur correspond au diamètre intérieur du trou traversant (28) soit insérable dans le passage (24).

6. Prothèse selon l'une des revendications 1 à 3, **caractérisée en ce que** le passage (24) et le trou traversant (28) présentent une section transversale circulaire, dans laquelle le diamètre du passage (24) est supérieur au diamètre du trou traversant (28) et le trou traversant (28) présente sur son extrémité faisant face au passage (24) une section élargie (56) dont le diamètre correspond au diamètre du passage (24), de telle sorte que dans le passage (24), une douille de guidage (50) dont le diamètre intérieur correspond au diamètre intérieur du trou traversant (28) soit insérable dans la position de fonctionnement de telle sorte qu'elle vienne en prise avec une extrémité de prise (52) pour le blocage d'un mouvement d'articulation involontaire dans la section élargie (56).

7. Prothèse selon la revendication 5, **caractérisée en ce que** des moyens pour la fixation amovible de la douille de guidage (50) dans le passage (24) sont prévus sur la douille de guidage (50) et le passage (24).

8. Prothèse selon la revendication 6, **caractérisée en ce que** des moyens pour la fixation amovible de la douille de guidage (50) dans le trou traversant (28) sont prévus sur la douille de guidage (50) et le trou traversant (28).

9. Prothèse selon l'une des revendications précédentes 3 à 8, **caractérisée en ce que** dans le passage (24) est disposé amovible un bouchon de fermeture (70) dont la surface extérieure affleure la surface du composant de prothèse fémoral.

10. Prothèse selon la revendication 9, **caractérisée en ce que** le bouchon de fermeture (70) est constitué de PTFE, de PVC ou de PEEK.

11. Prothèse selon l'une des revendications précédentes, **caractérisée en ce que** dans l'élément de prothèse tibial (14) est ménagé au moins un trou transversal latéral (34) à travers lequel des vis transversales ou des boulons transversaux sont insérables pour la liaison par coopération de formes de l'élément de prothèse tibial (14) avec une pièce d'ancrage de manche (32), un clou intramédullaire d'allongement (80) et/ou le tibia (102).

12. Prothèse selon l'une des revendications précédentes, **caractérisée en ce que** dans l'élément de prothèse tibial (14) est prévue une ouverture latérale (86) pour la mise en oeuvre d'un câble (88) pouvant être relié à un clou intramédullaire d'allongement (80).

13. Prothèse selon l'une des revendications précédentes, **caractérisée en ce que** le canal de guidage (42) passe à travers le dispositif d'articulation (30).

14. Prothèse selon l'une des revendications 1 à 12, **caractérisée en ce que** le canal de guidage (42) est disposé en dehors du dispositif d'articulation (30).

15. Prothèse selon l'une des revendications 3 à 14, **caractérisée en ce que** l'élément de prothèse fémoral (12) forme une pièce avec la tête d'articulation (22) et/ou l'élément de prothèse tibial (14) forme une pièce avec l'élément d'articulation (26).
